# EUROPEAN PATENT SPECIFICATION

(11) **EP 3 107 573 B1**
(45) Date of publication and mention of the grant of the patent: **26.09.2018**
(21) Application number: 15705772.0
(22) Date of filing: 18.02.2015
(51) Int. Cl.: A61K 39/395, A61K 39/39

(54) **CANCER-TARGETED IL-12 IMMUNOTHERAPY**
IL-12-BASIERTE IMMUNTHERAPIE GEGEN KREBS
IMMUNOTHÉRAPIE DU CANCER PAR IL-2

(30) Priority: 19.02.2014 EP 14000585
(43) Date of publication of application: 28.12.2016
(73) Proprietor: Merck Patent GmbH, 64293 Darmstadt (DE)
(72) Inventor: STRITTMATTER, Wolfgang, 64372 Ober-Ramstadt (DE); HANDGRETINGER, Rupert, 72076 Tübingen (DE); SCHILBACH-STUECKLE, Karin, 72074 Tuebingen (DE)
(86) International application number: PCT/EP2015/000363
(87) International publication number: WO 2015/124297

(56) References cited:
- WO-A1-2010/046097
- FALLON JONATHAN ET AL: "The immunocytokine NHS-IL12 as a potential cancer therapeutic", ONCOTARGET, vol. 5, no. 7, April 2014 (2014-04), pages 1869-1884, XP55190573,
- DAVIS C B ET AL: "Immunocytokines: Amplification of anti-cancer immunity", CANCER IMMUNOLOGY, IMMUNOTHERAPY 20030501 DE, vol. 52, no. 5, 1 May 2003 (2003-05-01), pages 297-308, XP2350474, ISSN: 0340-7004
- GRANGE M ET AL: "Activated STAT5 promotes long-lived cytotoxic CD8<+>T cells that induce regression of autochthonous melanoma", CANCER RESEARCH 20120101 AMERICAN ASSOCIATION FOR CANCER RESEARCH INC. USA, vol. 72, no. 1, 1 January 2012 (2012-01-01), pages 76-87, XP002739880, ISSN: 0008-5472

## Description

### FIELD OF THE INVENTION:

The invention is directed to cancer immunotherapy. The invention is specifically directed to the induction of innate or adaptive antitumor immunity initiated by the administration of targeted IL-12 molecules preferably in conjunction with IL-2 and / or IL-7 to a cancer patient, who suffers from cancer of the muscle, bone, nerves, cartilage, tendons, blood vessels, etc., preferably from sarcoma.

The invention is specifically related to the use of IL-12 in form of the specific immunoglobulin cytokine fusion protein called NHS-IL12, preferably in combination with a form of IL-7 exhibiting prolonged pharmacokinetics for the treatment of said cancer diseases, specifically sarcomas.

### BACKGROUND OF THE INVENTION

Cancer immunotherapy encompasses a diverse variety of treatment approaches including 'passive' administration of tumor-specific monoclonal antibodies and other immune system components, 'active' immunization to elicit or augment specific T cell-mediated immune responses against tumor cells, adoptive transfer of ex vivo modified T cells, and non-specific enhancement of immune responsiveness with immune modulatory agents. Immunotherapy has already had a major impact on the management of a broad range of cancers, but this has been largely restricted to passive immunotherapy with monoclonal antibodies. The field of cancer immunotherapy is complex and rapidly evolving. Immunotherapies differ from conventional chemotherapy in their mechanisms of action as well as the types of responses produced, and conventional response criteria may not provide a reliable assessment of the disease-modifying activity of immunotherapeutic agents. Many active immunotherapies incorporate multiple components (e.g. antigens, adjuvants and delivery vehicles). Furthermore, it is increasingly recognized that robust therapeutic activity requires that immunization is combined with other immunomodulatory strategies directed at enhancing general immune responsiveness and overcoming the immunosuppressive mechanisms through which tumours promote a tolerogenic environment.

Rhabdomyosarcoma (RMS) is the most common soft tissue tumor in children and is associated with a very unfavorable prognosis in advanced *stage (*Oberlin et al., 2008, J. Clin. Oncol. 26:2384-2389*).* Surgical resection, chemo- and radiotherapy often fail due to tumor localization in delicate anatomical sites and propensity for spreading. Thus, there exists a high unmet need for alternative treatment strategies.

Recent data suggest allogeneic stem cell transplantation (alloSCT) as a feasible strategy in advanced solid tumors, including RMS *(*Koscielniak et al., 2005, J. Clin. Oncol. 23:242-244*).* Yet, the mechanisms of tumor control in this setting have not been well elucidated. Although cytotoxic T- and natural killer (NK)-cell responses against RMS can be generated *in vitro (*van den Broeke et al., 2006, Cancer Res. 66:1818-1823*),* antitumor activity of immune effector cells has many facets. Beyond killing, combined signaling through interferon-gamma (IFN-y) and tumor necrosis factor receptor1 (TNFR1)-dependent pathways can result in tumor dormancy *(*Muller-Hermelink, et al 2008, Cancer Cell 13:507-518*),* thus, the induced secretion of these cytokines may be an important way to locally control tumor growth. Indeed, several studies have shown improved survival of patients with increased frequencies of IFN-γ producing T cells after alloSCT *(*Wiegering et al., 2011, Cancer Immunol. Immunother. 60:693-703*).* Thereby, CD4⁺ T cells may be as important as CD8⁺ CTLs.

IL-12, a major player in this network, can induce tumor regression and impacts innate and adaptive immunity *(e.g.* Trinchieri,G. 2003, Nat. Rev. Immunol. 3:133-146*).* Besides its role in T-cell priming, IL-12 reverts T_{H}17 cells back into T_{H}1 phenotype, restores M1 macrophage function and mediates DC-NK interactions.

Interleukin-12 (IL-12) is a pleiotropic proinflammatory cytokine that is produced in response to infection by a variety of cells of the immune system, including phagocytic cells, B cells and activated dendritic cells (Colombo and Trinchieri (2002), Cytokine & Growth Factor Reviews, 13: 155-168). IL-12 plays an essential role in mediating the interaction of the innate and adaptive arms of the immune system, acting on T-cells and natural killer (NK) cells, enhancing the proliferation and activity of cytotoxic lymphocytes and the production of other inflammatory cytokines, especially interferon-γ. IL-12 is a heterodimeric molecule composed of an α-chain (the p35 subunit, IL-12p35) and a β-chain (the p40 subunit, IL-12p40) covalently linked by a disulfide bridge to form the biologically active 74 kDa heterodimer.

The presence of endogenous IL-12 has been shown to be necessary for immunological resistance to a broad array of pathogens, as well as to transplanted and chemically induced tumors (Gateley et al. (1998), Annu. Rev. Immunol., 16: 495-521). IL-12 has been demonstrated to have a potent anti-tumor activity based upon the induction of IFN-γ and the activation of effector cells such as CD8+ T-cells and NK cells (Brunda et al. (1993), J. Exp. Med., 178: 1223-30). High levels of IFN-gamma are produced by T cells and NK cells in response to IL-12 *[*Kobayashi et al., 1989, J Exp Med;170:827-45*],* leading to enhanced antigen-presentation through paracrine upregulation of MHC class I and class II expression Wallach et al., 1982 Nature 1982;299:833-69]. As a result of its demonstrated anti-tumor activity, IL-12 has been tested in human clinical trials as an immunotherapeutic agent for the treatment of a wide variety of cancers (Atkins et al. (1997), Clin. Cancer Res., 3: 409-17; Gollob et al. (2000), Clin. Cancer Res., 6: 1678-92; and Hurteau et al. (2001), Gynecol. Oncol., 82: 7-10), including renal cancer, colon cancer, ovarian cancer, melanoma and T-cell lymphoma, and as an adjuvant for cancer vaccines (Lee et al. (2001), J. Clin. Oncol. 19: 3836-47).

Systemic administration of IL-12 has principally shown efficacy against some solid tumors but its use in therapy is limited because of its dose-limiting toxicity *(*Gollob et al., 2000, Clin. Cancer Res. 6:1678-1692*).* After over a decade in early clinical development, documented cases of severe toxicity and generally low response rates to rIL-12 have prevented its clinical development so far. WO2010/046097 A1 discloses cancer treatments with radiation and tumor-targeted NHS-IL-12.

Thus, the targeted delivery of IL-12 to the tumor microenvironment represents a highly promising approach for tumor immunotherapy, because it could render the cytokine more effective and less toxic. Therefore, it is the major subject of this invention, to provide an effective and above all exercisable therapeutic cancer-immunotherapy approach by using the efficient antitumor immunity of the drug.

### SUMMARY OF THE INVENTION

One strategy for improving the safety of proinflammatory cytokines, such as IL-2 and IL-12, is to direct their delivery to tumors via fusion to a tumor-targeting antibody. Such antibody-cytokine fusion proteins, or "immunocytokines," have previously demonstrated the ability to enhance anti-tumor immunity in preclinical *models (*Gillies SD. In Lustgarten J, Cui Y, Li S, eds. Targeted Cancer Immune Therapy. New York, New York, USA: Springer; 2009:241-256)*.* To maximize immunocytokine tolerability, the antibody selected as a vehicle must bind specifically to an antigen uniquely found in tumors. Antibodies directed against necrosis-associated antigens, which are abundantly present in tumors but not in normal tissues, offer an attractive delivery approach (for example, Epstein et al., 1988, Cancer Res 1988;48:5842-48).

The ability of DNA/histone-binding antibodies to selectively target to regions of tumor necrosis has been well studied both preclinically and clinically. Taking advantage of this concept, a necrosis-targeted IL-12 immunocytokine, called NHS-IL12, was engineered by genetically fusing 2 human IL-12 heterodimers to the C-termini of the heavy chains of the NHS76 antibody (WO 2000/001822). NHS76 is a fully human, phage display-derived IgG1 antibody selected for its specific ability to bind to DNA/histones and thereby target to tumors *in vivo* [Sharifi et al., 2001, Hybrid Hybridomics;20:305-12*]*.

It was found that the IL-12-driven human immune system, by administering the above-specified targeted IL-12 drug does not only kill cancer cells but also uses alternative mechanisms to attenuate cancer growth, i.e. by inducing senescence and/or differentiation in cancer cells, thus leading to remission of cancer cells or cancer tissue to cells or tissue of origin. The proposed targeted IL12 therapy causes specifically T_{H}1-induced growth arrest and differentiation of cancer cells. The effect is specifically increased if the targeted IL-12 is administered in conjunction or combination with immune modulating agents, such as interleukins, for example IL-2 and IL-7.

IL-12 is used in a bound and targeted form, such as an IL-12-fusion protein, preferably a fusion protein composed of an immunoglobulin that is able to target an antigen or a specific receptor molecule expressed by the patient's tissue, and IL-12, wherein preferably the C terminus of the immunoglobulin is linked to the N-terminus of the immunocytokine. Preferably, the therapeutically effective antibody or targeting portion thereof is directed to DNA-histone H1 complex exposed in tumor necrosis. In a further specific embodiment the targeted IL-12 molecule is a respective fusion protein composed of IL-12 and known fully human IgG1 antibody NHS76, described above and below in detail.

The targeted IL-12 as disclosed herein can be used in cancer immunotherapy. According to the invention, the targeted IL-12 can be used in cancer immunotherapy, wherein the cancer is related to muscles, bones, nerves, cartilages, tendons, blood vessels, etc., preferably sarcoma, and in more preferably, rhabdomyosarcoma (RMS). According to the invention the targeted IL-12 can be used in monotherapy or in combination with immune-modulating agents, such as interleukins, interferons, CpGs, chemokines or glucans.

In a specific embodiment the immune modulation agent is IL-7. In a further specific and preferred embodiment the immune modulation agent is IL-2. The immune modulating agent is preferably covalently bound or fused to a larger protein or immunoglobulin or a fragment thereof (such as Fab, scFv, Fc) or is in complex with an immunoglobulin. In one embodiment of the invention IL-7 is covalently fused to an Fc portion of an antibody.

The invention is further directed to tumor-targeted IL-12 for use in a combination treatment with an immune modulating and / or immune complementary agent, such as an interleukin, interferon or chemokine, for inducing and / or stimulating the immune response against a cancer disease in a patient suffering from said cancer disease, wherein said induction or stimulation causes senescence of cancer cells, and remission of cancer cells or cancer tissue to cells or tissue of origin, wherein said the tumor-targeted IL-12 is a therapeutically effective monoclonal antibody or a biologically active portion thereof, directed to human DNA-histone H1 complex exposed in tumor necrosis, and fused via its C-terminus to IL-12.

The tumor-targeted IL-12 therapy as described herein is useful to cause cancer cell senescence, which is preferably generated by increased production of endogenous IFNy and / or TNF in succession of said stimulation or induction of the patient's immune system triggered by said targeted IL-12 therapy. It was found that by said treatment according to the invention the senescence of the cancer cells results in stable growth arrest. Furthermore it was found that the senescence of the cancer cells and tissue remission is independent on direct immune specific cytotoxic effects, such as generation and activation of NK-cells and macrophages, although these cells are triggered by IL-12.

The tumor-targeted IL-12 therapy as disclosed herein is useful in cancer immunotherapy, wherein the cancer disease is related to solid tumors, or tumors of the muscle, bone, nerves, cartilage, tendons, blood vessels, and fatty or fibrous tissues. In a specific embodiment of the invention the therapy is used for treating sarcoma, and preferably rhabdomyosarcoma (RMS).

It was found that the proposed cancer immunotherapy, wherein NHS-IL12 and IL-7 as specified above and below are used combination, does not only provoke senescence of cancer cells but in addition initiates induction of myogenic differentiation and remission of cancer cells to tissue cells of origin, indicating that the IL-12-driven human immune system does not only kill cancer cells but also uses alternative mechanisms to attenuate cancer growth. Tumor-targeted IL-12 significantly abrogates cancer growth at least in NSG mice reconstituted with a fully humanized allogeneic immune system.

Neither IL-2 and IL-7 alone halted tumor growth nor induced an effective anti-tumor immune response. Stimulation of the allogeneic human immune system with IL-12 is needed to efficiently control tumor growth in humanized mice. More efficiently in this context is the combination of NHS-IL12 and IL-7, above all, when IL-7 is in a form such that its half-life in the body is increased. In one embodiment, IL-7 is coupled or bound to an immunoglobulin, preferably IL-7 is fused to the C-terminus of the Fc fragment of an immunoglobulin.

The data as disclosed herein could lead to the suggestion that IL-12 promoted the allogeneic immune system to kill cancer cells, such as cancer cells of muscle, bone, nerves, cartilage, tendons, blood vessels, and fatty or fibrous tissues, preferably RMS cells at least in this particular system as used. Interestingly, immunohistology shows no evidence for cancer cell killing, apoptosis or necrosis, suggesting that targeted IL-12 cancer immunotherapy in combination with IL-7, preferably in bound or complex form, drives critical mechanisms of cancer control independent from killing. Most importantly however, cancers growth-arrested by administration of the targeted IL12 therapy according to the invention, start to *de novo* expression of desmin, a muscle-specific protein and a key subunit of the intermediate filament in cardiac, skeletal and smooth muscles, in steric cross-striated configuration. Especially the presence of desmin in a striated fashion points to a high degree of differentiation in muscle cells *(van,d.,* V, Schaart, et al., 1992, Cell Tissue Res. 270:189-1981*).*

In summary, the data provided by the inventors are the first *in vivo* evidence for T_{H}1-induced growth arrest and differentiation of cancer cells. This can be accomplished by the targeted delivery of IL-12, preferably in combination with IL-7, to the tumor microenvironment and the subsequent activation of the p16^{INK4a} pathway.

### DETAILED DESCRIPTION

The term *"senescence"* means according to the invention a growth-arrest program that limits the lifespan of cancer cells and prevents unlimited cell proliferation.

The term *"direct immune specific cytotoxic effects"* means according to the invention the active involvement and action of immune specific cytotoxic cells, such as NK cells macrophages, T-cytotoxic cells, or dendritic cells during immune response.

The term "cancer immunotherapy" means according to the invention a therapeutic treatment that stimulates or restores the ability of the immune system to fight cancer by inducing, enhancing or suppressing an immune response. Cancer immunotherapy results in targeted immune activity against a disease-specific antigen, either by increasing immune cell recognition of the target or by reducing disease-related immune suppression.

According to the invention, it is preferred according to the invention to combine the administration of targeted IL-12, such as NHS-IL12, with IL-7, preferably with IL-7 in bound, fused or complex form as specified in detail above and below. The co-administration can be done simultaneously or sequentially, wherein in the latter case the immunomodulating agent can be administered hours or days before or after administration of the targeted IL-12 molecule like NHS-IL12, following a specific dose and time regimen.

According to the invention, it is principle possible to combine the administration of targeted IL-12, such as NHS-IL12 with radiotherapy and/ or chemotherapy.

The chemotherapeutic agent used in combination with above-said targeted IL-12 fusion molecules according to the invention may be e.g. methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine, UFT(Tegafur/Uracil), ZD 9331, Taxotere/Decetaxel, Fluorouracil (5-FU), vinblastine, and other well compounds from this class. Chemotherapy is applied according to the invention by at least two cycles, preferably 2 - 8 cycles, more preferably 2 - 5 cycles. One cycle is between 21 and 35 days, preferably between 21 - 28 days. The dose regimen of the chemotherapeutic agent is dependent on various possible patient- and drug-related conditions and properties..

Radiotherapy is carried out according to the invention by standard radiation, wherein a total of 40 - 120 Gy are applied, preferably at least 50 Gy, more preferably between 50 and 75 Gy. The radiation therapy is usually fractionated, wherein 1.5 - 3.5 Gy are applied per day for at least four days, preferably 5 - 7 days in sequence. The total radiation dose is to be applied according to the invention within 21 - 35 days, preferably within 28 days. If necessary or favourable, boost doses of 3.5 - 15 Gy, preferably 5 - 10 Gy can be applied at the beginning of radiation or in an intermediate interval. Radiotherapy can be carried out according to the invention before or after administration with the targeted IL-12 molecules and optionally the immune modulation agents of the invention, or simultaneously.

The chemo-radiotherapy treatment can be accompanied by administration of an agents that is capable to modulate the immune system. By, for example, applying a relatively low dose of cyclophosphamide between 100 - 400 mg/m²preferably 250 mg/m² the immune system of the patient can be activated or enhanced. Usually, a single dose before start of the vaccination, as a rule 1 to 5 days, preferably 2 - 5 days, should be sufficient to be effective.

The preferred targeted IL-12is, as specified above, NHS-IL12. As mentioned above, IL-12 is a heterodimeric molecule composed of an α-chain (the p35 subunit, IL-12p35) and a β-chain (the p40 subunit, IL-12p40) covalently linked by a disulfide bridge. Within the fusion protein of the invention the p35 subunit is linked to the C-terminus of each of the two heavy chains of the (dimeric) antibody NHS76. The p40 subunit is linked to the p35 subunit by covalent binding. In practice, the molecule is manufactured by recombinant methods using a DNA construct expressing the heavy chain and the p35 subunit as fusion protein and separately the p40 subunit, which binds in situ to the expressed NHS-p35 subunit fusion as described.
SEQ ID NO.1 depicts the mature amino acid sequence of the α chain, *i.e.,* the human p35 subunit, of a mature (wild-type) human IL-12: 
RNLPVATPDPGMFPCLHHSQNLLRAVSNMLQKARQTLEFYPCTSEEIDHEDITKDKTSTVEACLPLELTKNESCLN
SRETSFITNGSCLASRKTSFMMALCLSSIYEDLKMYQVEFKTMNAKLLMDPKRQIFLDQNMLAVIDELMQALNFNS
ETVPQKSSLEEPDFYKTKIKLCILLHAFRIRAVTIDRVMSYLNAS
SEQ ID NO. 2 depicts the mature amino acid sequence of the β chain, *i.e.,* the human p40 subunit, of a mature (wild-type) human IL-12: 
IWELKKDVYVVELDWYPDAPGEMVVLTCDTPEEDGITWTLDQSSEVLGSGKTLTIQVKEFGDAGQYTCHKGGEVLS
HSLLLLHKKEDGIWSTDILKDQKEPKNKTFLRCEAKNYSGRFTCWWLTTISTDLTFSVKSSRGSSDPQGVTCGAAT
LSAERVRGDNKEYEYSVECQEDSACPAAEESLPIEVMVDAVHKLKYENYTSSFFIRDIIKPDPPKNLQLKPLKNSR
QVEVSWEYPDTWSTPHSYFSLTFCVQVQGKSKREKKDRVFTDKTSATVICRKNASISVRAQDRYYSSSWSEWASVP
CS
SEQ ID NO. 3 depicts the amino acid sequence of the lambda light chain of Mab NHS as used and modified according to the invention including signal sequence (italics, first 19 aa), variable domain (underlined), and de-immunizing L103V mutation (in bold). The signal sequence is not part of the mature polypeptide chain: 
MELPVRLLVLMFWIPASLSSSELTQDPAVSVALGQTVRITCQGDSLRSYYASWYQQKPGQAPVLVIYGKNNRPSGI
PDRFSGSSSGNTASLTITGAQAEDEADYYCNSRDSSGNHVVFGGGTKVTVLGQPKAAPSVTLFPPSSEELQANKAT
LVCLISDFYPGAVTVAWKADSSPVKAGVETTTPSKQSNNKYAASSYLSLTPEQWKSHKSYSCQVTHEGSTVEKTVA
PTECS
SEQ ID NO. 4 depicts the amino acid sequence of the heavy chain / human p35 fusion, including signal sequence (italics, first 19 aa), variable domain (underlined), and R to A substitution of first amino acid of hu p35(in bold), hu p35 (underlined and italicized). The signal sequence is not part of the mature polypeptide chain: 
MELPVRLLVLMFWIPASLSQVQLQESGPGLVKPSETLSLTCAVSGYSISSGYYWGWIRQPPGKGLEWIGSIYHSGS
TYYNPSLKSRVTISVDTSKNQFSLKLSSVTAADTAVYYCARGKWSKFDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQGNVFSCSVMHEALHNHYT
QKSATATPGAANLPVATPDPGMFPCLHHSQNLLRAVSNMLQKARQTLEFYPCTSEEIDHEDITKDKTSTVEACLP
LELTKNESCLNSRETSFITNGSCLASRKTSFMMALCLSSIYEDLKMYQVEFKTMNAKLLMDPKRQIFLDQNMLAVI
DELMQALNFNSETVPQKSSLEEPDFYKTKIKLCILLHAFRIRAVTIDRVMSYLNAS
SEQ ID NO. 5 depicts the amino acid sequence of human p40, with signal sequence (italicized) and variant sequence shown in bold (KSKREKKDRV mutated to KDNTEGRV). The signal sequence is not part of the mature polypeptide chain:

MCHQQLVISWFSLVFLASPLVAIWELKKDVYVVELDWYPDAPGEMVVLTCDTPEEDGITWTLDQSSEVL
GSGKTLTIQVKEFGDAGQYTCHKGGEVLSHSLLLLHKKEDGIWSTDILKDQKEPKNKTFLRCEAKNYSG
RFTCWWLTTISTDLTFSVKSSRGSSDPQGVTCGAATLSAERVRGDNKEYEYSVECQEDSACPAAEESLP

IEVMVDAVHKLKYENYTSSFFIRDIIKPDPPKNLQLKPLKNSRQVEVSWEYPDTWSTPHSYFSLTFCVQ
VQGKDNTEGRVFTDKTSATVICRKNASISVRAQDRYYSSSWSEWASVPCS

### EXAMPLES

### Establishing a fully functional immune system in NSG mice

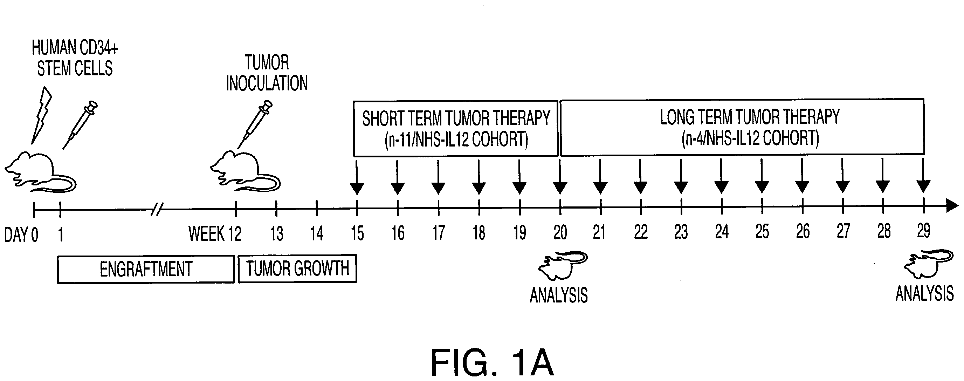

To test the toxicity and potential therapeutic efficacy of the NHS-IL12 construct in the human system *in vivo,* NSG mice were transplanted with huCD34⁺ stem cells and stimulated engraftment with FcIL-7 (Figure 1A). Transplantation of >99.9% pure huCD34⁺ cells into NSG mice established all hematopoietic lineages within 12 weeks. In addition, complex T-cell receptor (TCR) repertoires were found in bone marrow, thymus, and spleen and thymus equivalents. Together, the data show that the transplanted mice developed a human T-cell immune system that closely resembled a human *in vivo* situation. The mice also developed normal NK cells, as shown by NKp receptors and killer-cell immunoglobulin-like receptors (KIR) that reflected the donor's NK repertoire.

### Tumor inoculation

Subcutaneous inoculation of 1x10⁶ allogeneic A204 cells 12 weeks post stem-cell transplantation resulted in aggressively growing tumors in all mice within 3 weeks. In line with stem-cell transplantation (SCT) of patients with RMS, the immune system failed to reject the allogeneic A204. The tumors grew rapidly, despite solid expression of surface HLA class I and II, MICA/B, Nectin-2 (CD112) and poliovirus receptor (PVR, CD155) but completely lacked UL16-binding proteins (ULBP) 1-4.

### Sarcoma therapy with histone-targeted IL-12 fusion proteins

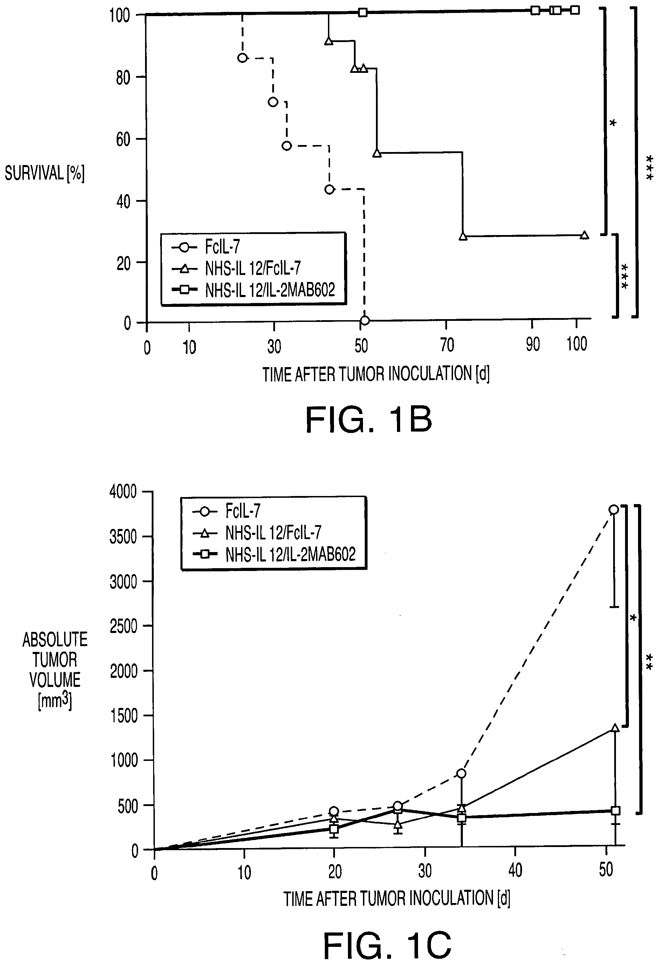

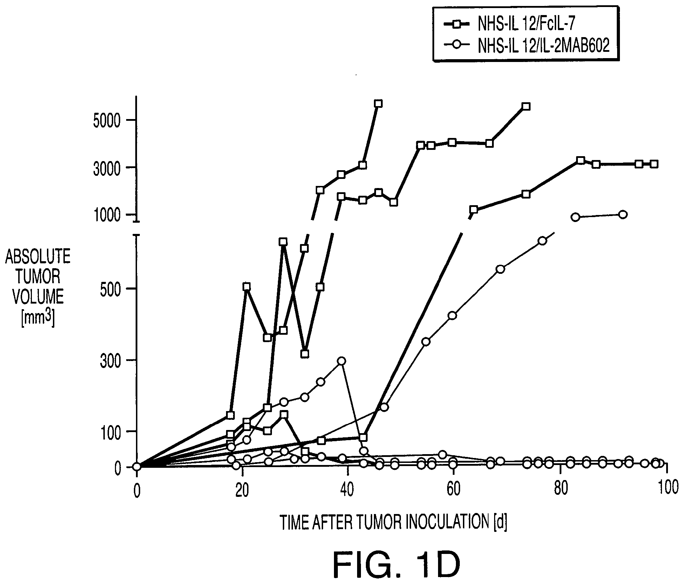

Following tumor inoculation, solid A204 RMS tumors became established after 3 weeks. Subsequently, mice were treated weekly for 5 weeks with either FcIL-7 alone (control), NHS-IL12/FcIL-7 or NHS-IL12/IL-2MAB602 (Figure 1A). Intravenous injection of the constructs caused no visible systemic toxicity, neither acutely nor over time (Figure 1B: 4 mice/cohort, 100 days). In mice treated with FcIL-7 only, sarcomas showed exponential growth. 4/7 mice died before week 5, and 3 mice reached endpoint criteria due to sarcoma burden at day 52 (Figure 1C). In the Fc-IL7 group, 6.5-fold tumor growth was observed from day 25 to day 52, whereas in the NHS-IL12/IL-2MAB602 the growth was reduced to 1.8-fold (*P*<0.05, one-tailed *t*-test). Thus, the sarcomas remained significantly growth-arrested in mice receiving NHS-IL12/IL-2MAB602 (n=11) (Figure 1C), resulting in the survival of all miceved (Figure 1B and 1D). The NHS-IL12/FcIL-7 protected 2/11 mice only for a shorter period of time (Figure 1B and 1C), as they died on day 43 and day 49 respectively. To get analysis from all 3 groups, mice were sacrificed on day 52 (short-term treatment), except for 4 mice per NHS-IL12 treatment group, that were kept alive and received therapy until day 100 (long-term treatment). NHS-IL12/FcIL-7 long-term treatment successfully halted tumor growth in 1/4, delayed tumor growth in 2/4 and eliminated the tumor in 1 mouse. NHS-IL12/IL-2MAB602 long-term treatment eliminated tumors in 3/4 mice and halted tumor growth in the remaining mouse (Figure 1D).

### Biodistribution of the NHS-IL12 construct

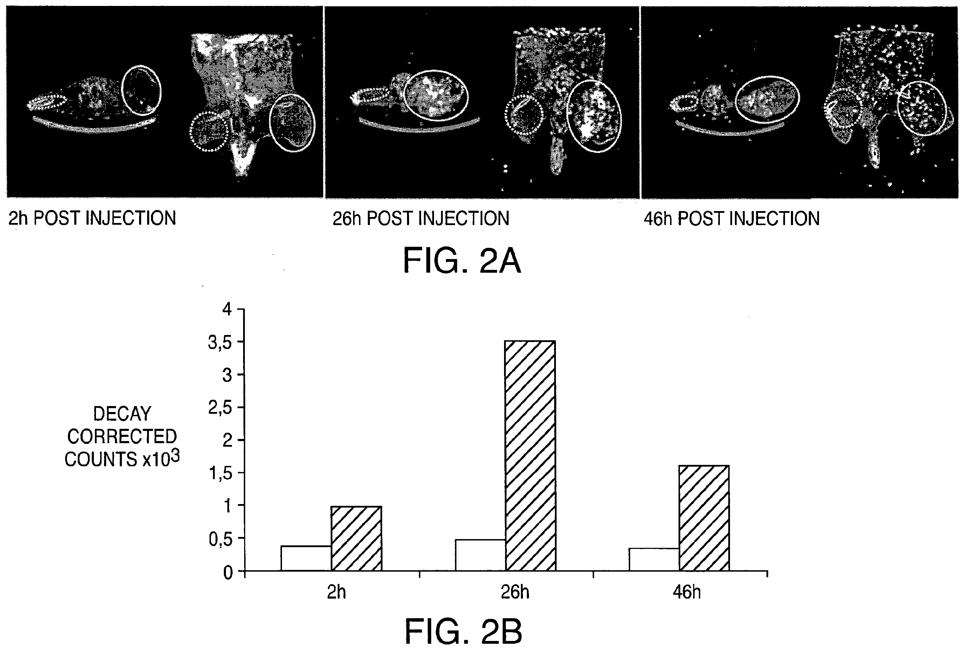

The monoclonal antibody NHS76 recognizes an intracellular antigen in necrotic cancer regions. It was therefore analyzed, whether NHS-IL12 binds preferentially to sites of sarcomas. SPECT/CT biodistribution studies with ¹²³I-labelled NHS-IL12 revealed significant *in vivo* enrichment of NHS-IL12 inside the sarcoma microenvironment (Figure 2A). Quantification of ¹²³I-labelled NHS-IL12 showed a four- to six-fold radionuclide enrichment in the tumors as compared with the contralateral muscle. ¹²³I counts peaked in the tumor region 26 h after intravenous NHS-IL12 application, whereas in normal muscle tissue ¹²³I counts remained stable over time (Figure 2B), confirming that NHS-IL12 preferentially bound to human sarcoma.

### Tumor-specific immune responses

To understand the differences underlying the therapeutic efficacy of the different treatment protocols, the inventors performed histology, immunohistochemistry (IHC) and extensive molecular and functional characterization of the human immune cells infiltrating the A204 sarcomas.

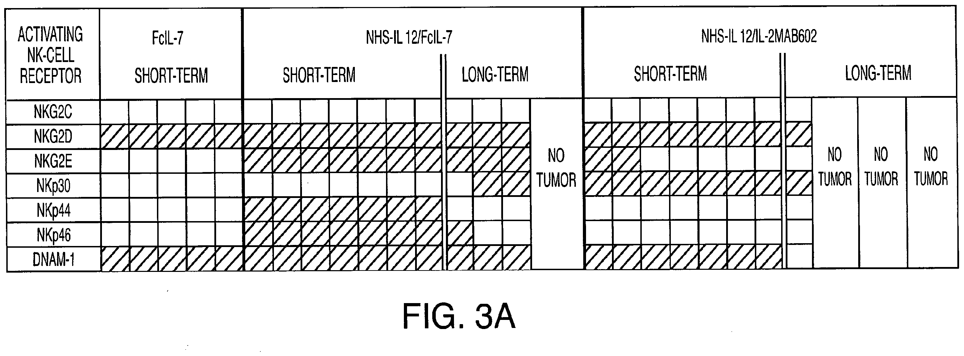

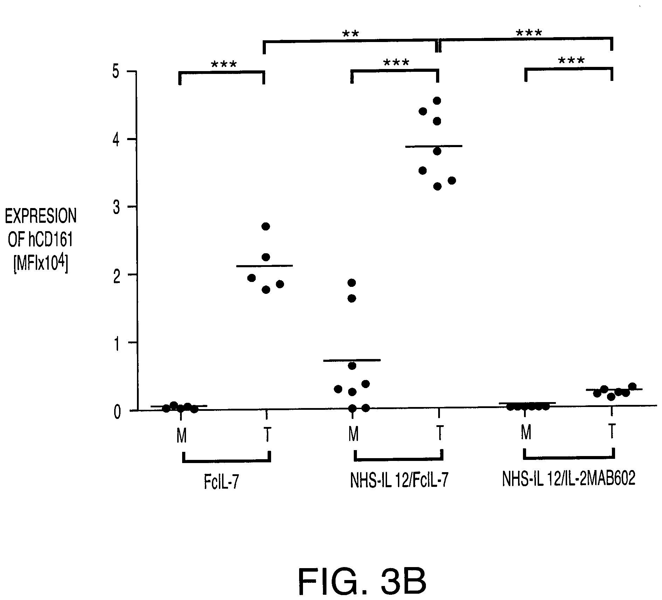

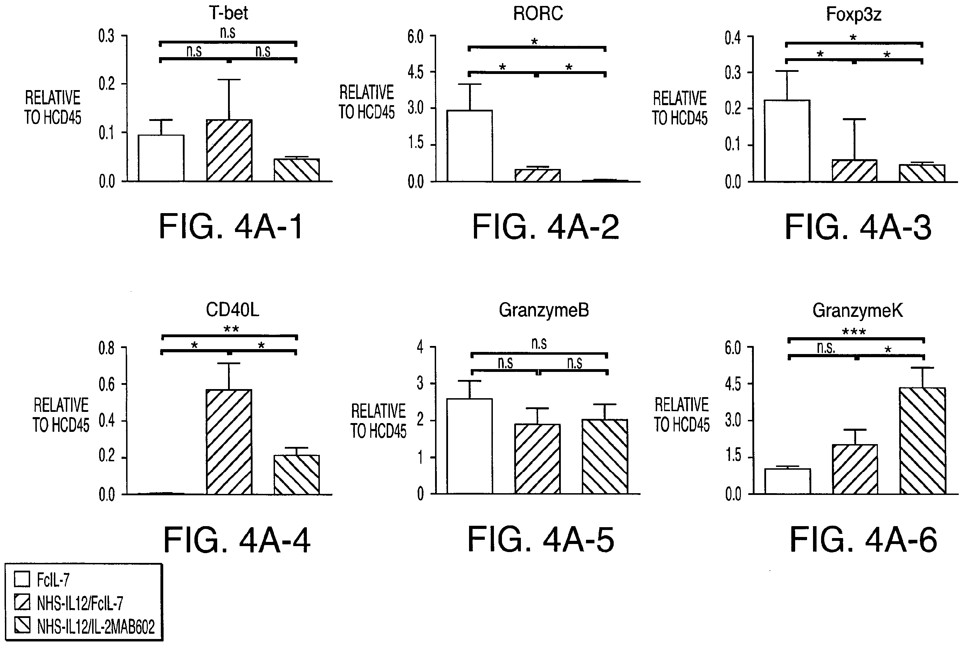

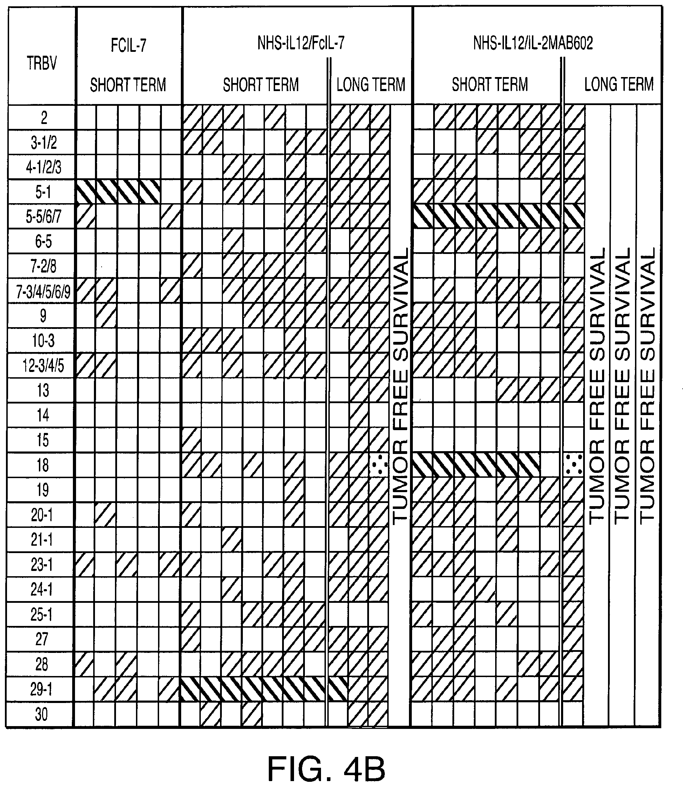

Strikingly, sarcomas of FcIL-7-treated mice had only a minor infiltrate, containing exclusively macrophages (CD68⁺) and NK cells (CD56⁺). In sharp contrast, sarcomas of mice treated with either NHS-IL12 regimen showed a dense mononuclear infiltrate with NK cells, macrophages and large numbers of CD4⁺ and CD8⁺ T cells. The NK cells of all treatment groups expressed NKG2D mRNA and DNAM-1 (Figure 3A), a ligand for the sarcoma-associated surface molecules Nectin-2 (CD112) and PVR (CD155). mRNA expression of surface molecules steering NK-cell differentiation and activation strictly required the NHS-IL12 construct. FcIL-7 or IL-2MAB602 then modulated the effect of the NHS-IL12 construct on the infiltrating NK-cell population. NKG2E, NKp44, and NKp46 were found only in tumors of mice treated with NHS-IL12/FcIL-7, whereas NKp30 expression was restricted to sarcomas of NHS-IL12/IL-2MAB602-treated and NHS-IL12/FcIL-7 long-term treated mice (Figure 3A). Sarcomas of FcIL-7-treated mice strongly expressed CD161 (Figure 3B) and T_{H}17-master transcription factor RORC (Figure 4A), characterizing IL-17-producing phenotype (Billerbeck et al. (2010) Proc. Natl. Acad. Sci. USA. 107:3006-11). In sharp contrast NHS-IL12/FcIL-7-treated mice also had strong CD161 expression (Figure 4B) but significantly lower RORC expression (Figure 4A) characterizing effector and central memory T cells secreting high levels of IFN-γ and TNF but lacking lytic activity (Takahashi et al. (2006) J. Immunol 176:211-216). Mice treated with NHS-IL12/IL-2MAB602 were devoid of CD161 mRNA (Figure 3B) and RORC mRNA (Figure 4A) strongly suggesting that the IL-17-producing NK- or T-cell phenotypes were suppressed in these mice (Laurence et al. (2007) Immunity 26:371-381; Ghoreschi et al. (2010) Nature 467:967-971).

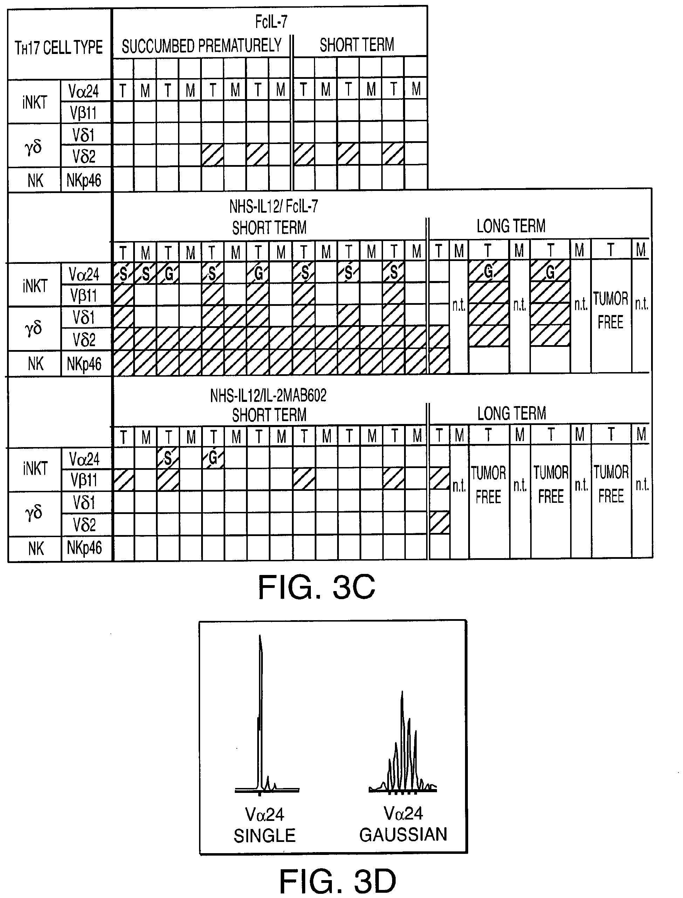

As KIR molecules impair NK-cell functions even in an MHC-I-deficient environment (27), KIR expression in sarcomas of either FcIL-7- or NHS-IL12/FcIL-7-treated mice was analyzed. qRT-PCR of total sarcoma revealed similar levels in both groups. As expected, KIR-expression of normal mouse muscle tissue homing NK cells and tumor infiltrating lymphocytes of human sarcoma xenografts differed, as mouse muscle showed KIR2DL3 and KIR2DL4, while the human sarcomas showed in addition KIR2DL1 and KIR3DL1. Despite the expression of the various KIR, NK cells remained functional, as, freshly isolated NK cells from sarcoma tissue released IFN-γ after *in vitro* stimulation with NHS-IL12. Besides NK cells, mRNA expression was found characterizing T_{H}17 innate lymphocyte populations, like TCRVα24-expressing iNKT cells, NKp46⁺ NK or γδ T cells almost exclusively in sarcomas of mice treated with NHS-IL12/FcIL-7 (Figure 3A, 3C, 3D).

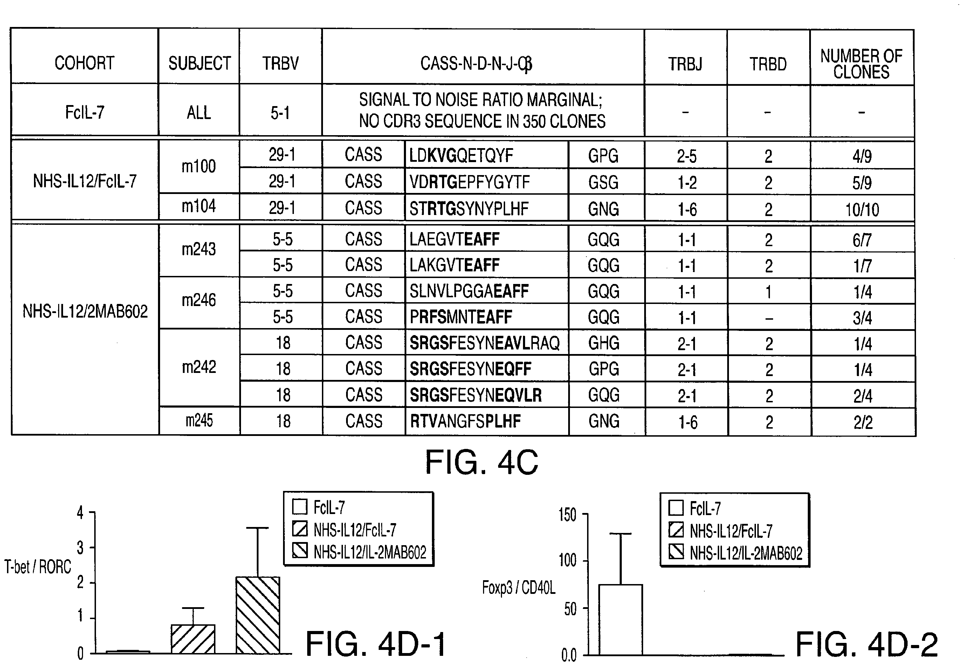

Tumors of all NHS-IL12-treated cohorts showed, besides innate lymphocyte populations, a broad spectrum of CD3⁺ T cells (Figure 4B and 4C). These were absent in sarcomas of mice treated with FcIL-7 that showed scarce signals in Vβ spectratype analysis (Figure 4B and 4C) and no infiltrating CD8⁺ T cells. NHS-IL12-treated sarcomas showed a broad TCR repertoire, substantiated by oligoclonal or monoclonal peaks within various Vβ-families (Figure 4C), as it occurs during preferential expansion of restricted T-cell clones. Cloning and sequencing of the CDR3 region confirmed that the peaks contained limited numbers of different T-cell clones with strongly amplified TRBV segments in the two treatment groups, such as TRBV29-1 in all individuals of the NHS-IL12/FcIL-7 cohort, or TRBV5-5 and TRBV18 in the NHS-IL12/IL-2MAB602 cohort (Figure 4B and 4C). The relative expression of transcription factors T-bet and RORC that regulate IFN-γ and IL-17 respectively mirrored the degree of T_{H}1 bias in the tumor infiltrating lymphocytes of the respective cohorts. The T-bet/RORC ratio was <0.05 in the FcIL-7-only cohort, while it was 19-fold higher (0.8) and 44-fold (2.2) higher in mice receiving NHS-IL12 with either Fc-IL7 or IL-2MAB602 (Figure 4D). In line with the T-bet/RORC expression, Foxp3 was about 10-fold lower in both NHS-IL12 groups than in the FcIL-7-only cohort (Figure 4A). Accordingly, low Foxp3 expression correlated inversely with a strong expression of the T-cell activation marker CD40L (Figure 4A and 4D).

### Senescence-induction in NHS-IL 12-treated sarcomas

The NHS-IL12 construct strongly suppressed sarcoma development in all treated mice (Figure 1). Surprisingly, only sarcomas of the NHS-IL12/IL-2MAB602-group contained high amounts of perforin protein and granzyme K mRNA (Figure 4A), while mice treated with NHS-IL12/FcIL-7 were virtually devoid of perforin protein and expressed low levels of granzyme K mRNA (Figure 4A). This strongly suggests that the sarcoma-controlling immune response included mechanisms different from cytolysis. Moreover, sarcomas did not contain sufficient numbers of CD4⁺ or CD8⁺ T cells to explain cancer control by killing or apoptosis.

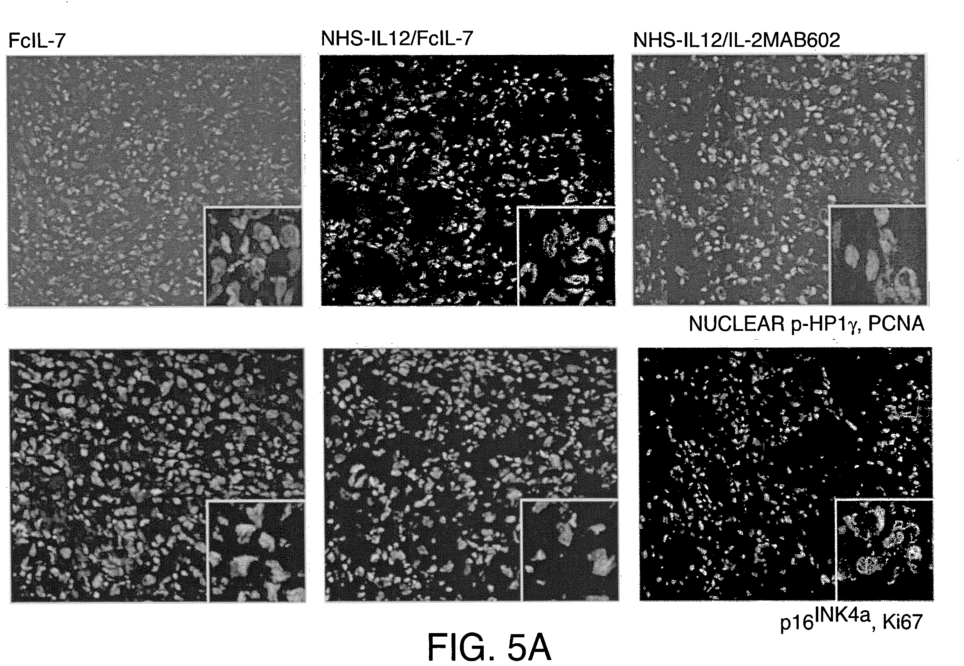

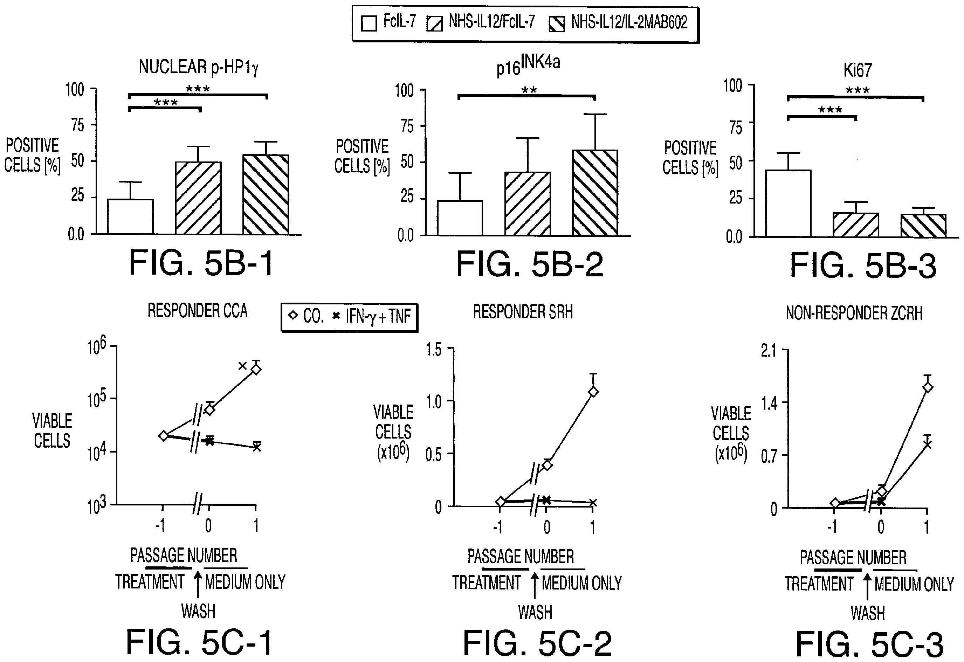

Therefore the effect of the immune response on sarcoma cell proliferation was analyzed, as determined by the proliferation marker proliferating cell nuclear antigen (PCNA) and Ki67. In the rapidly growing FcIL-7-control tumors 50% of the sarcoma cells stained positive for PCNA or Ki67, showing that most of the cells were proliferating (Figure 5A and 5B). In the NHS-IL12-treated groups, both PCNA- (Figure 5A) and Ki67- stained sarcoma cells were significantly lower than in the FcIL-7-control (Figure 5A and 5B). To determine whether the immune response just arrested the cell cycle or whether it induced a stable growth arrest as seen in cytokine-induced senescence, sarcomas were double stained for one proliferation marker and either senescence-associated phosphorylated heterochromatin protein 1 (p-HP1γ or p16^{INK4a}, also known as cell cycle regulator cyclin-dependent kinase inhibitor 2A (CDKN2A). FcIL-7-only treated sarcomas showed high PCNA/Ki67 expression and at the same time very low expression of p16^{INK4a}/nuclear p-HP-1γ, confirming that these sarcoma cells are rapidly proliferating (Figure 5A and 5B). In sharp contrast, up to 70% of the sarcoma cells from mice treated with either NHS-IL12/IL-2MAB602 or NHS-IL12/FcIL-7 expressed the senescence marker p-HP1γ or p16^{INK4a} (Figure 5A and 5B), in the absence of PCNA (PCNA⁻/p-HP1γ⁺) (Figure 5A, upper line inserts) and Ki67 (Ki67⁻/p16^{INK4a}) (Figure 5A, lower line inserts).

The data clearly show that IL-12-driven stimulation of the human immune system can use senescence in cancer cells as an important mechanism to constrain cancer growth.

Since IFN-γ and TNF are the two major effector cytokines of IL-12-driven T_{H}1-immunity and as these two cytokines can induce senescence, various patient-derived human RMS cell lines of very early passage were incubated with increasing doses of IFN-γ and TNF. Either cytokine alone caused no or only moderate growth inhibition. Yet, when combined they caused a permanent, senescence-defining growth arrest in 2 of 3 sarcomas (Figure 5C). Importantly, the senescence-resistant sarcoma did not express the cell cycle regulator p16^{INK4a} (not shown), confirming that IFN-γ and TNF-induced senescence strictly required the activation of p16^{INK4a}.

### Differentiation-induction in A204 rhabdomyosarcomas

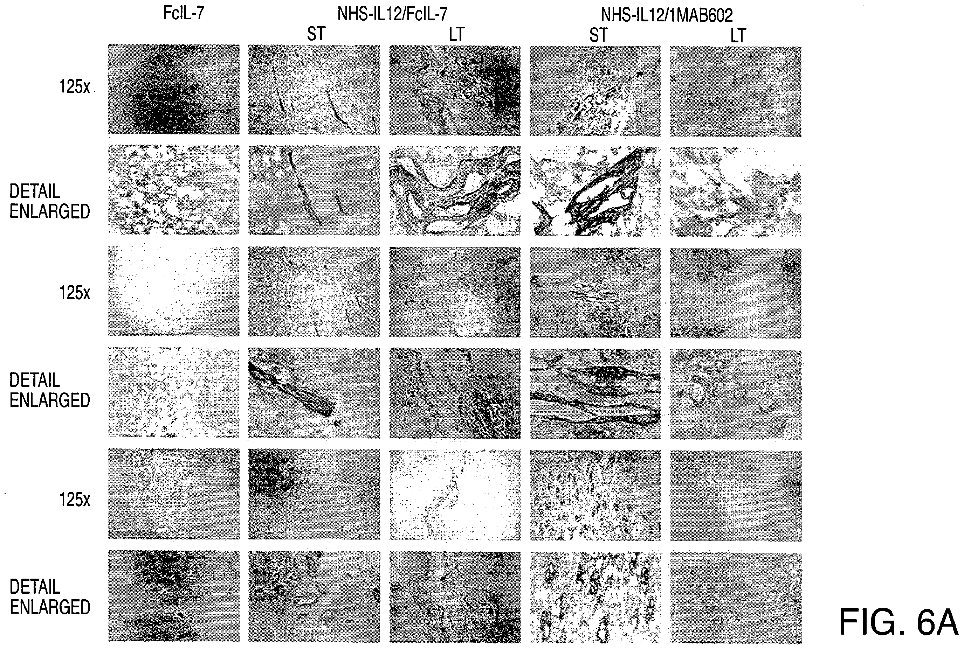

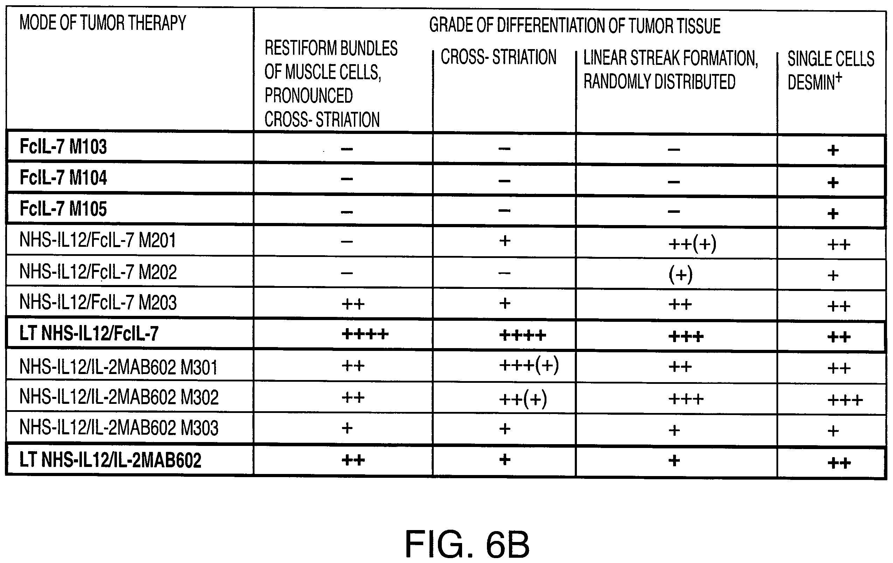

As IFN-γ- and TNF-dominated immune response caused permanent cell cycle arrest in the RMS, and skeletal muscle differentiation depends on the myoblasts' withdrawal from the cell cycle early to allow expression of muscle-specific genes and cell fusion into multinucleate myotubes, it is a matter of question whether this growth arrest might also affect the differentiation of A204 sarcomas. Desmin is a reliable marker for rhabdomyoblastic differentiation that is absent in either un- or poorly-differentiated RMS. Accordingly, A204 sarcomas showed neither the cross-striation that characterizes myocytes, nor did they express desmin prior to transplantation (not shown). Following transplantation into humanized mice, the FcIL-7-treated proliferating sarcomas remained poorly differentiated, with few single desmin⁺ cells diffusely distributed within the tumors but no cross-striation (Figure 6A and 6B). In sharp contrast, NHS-IL12 treatment did not only inhibit tumor growth and induced senescence, but also gradually restored the myogenic structure and marker expression in A204 sarcomas. NHS-IL12/FcIL-7 induced randomly distributed, linear areas of maturated RMS cells that clearly expressed desmin and areas with cross-striation. This differentiation towards functional muscle tissue was even more pronounced in sarcomas from mice treated with NHS-IL12/IL-2MAB602. Such growth-arrested sarcomas showed restiform / rope-like propagation of differentiation zones with cribriform / tube-like structures extensively penetrating the tumor (Figure 6A and 6B). Thus, growth arrest of sarcomas *in vivo* induced senescence and was associated with the restoration of the cell-fate specific markers of myocytes, the origin of A204 RMS.

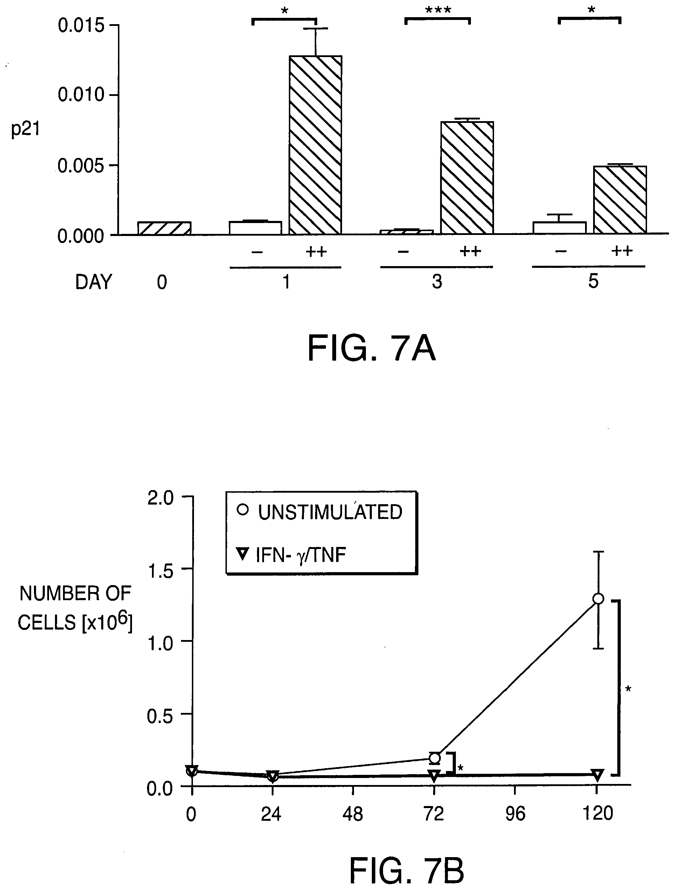

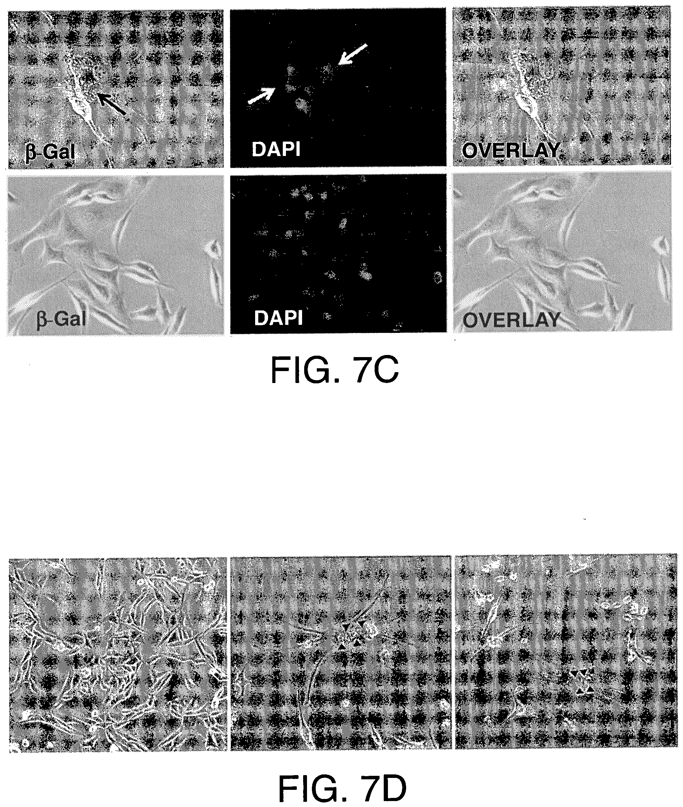

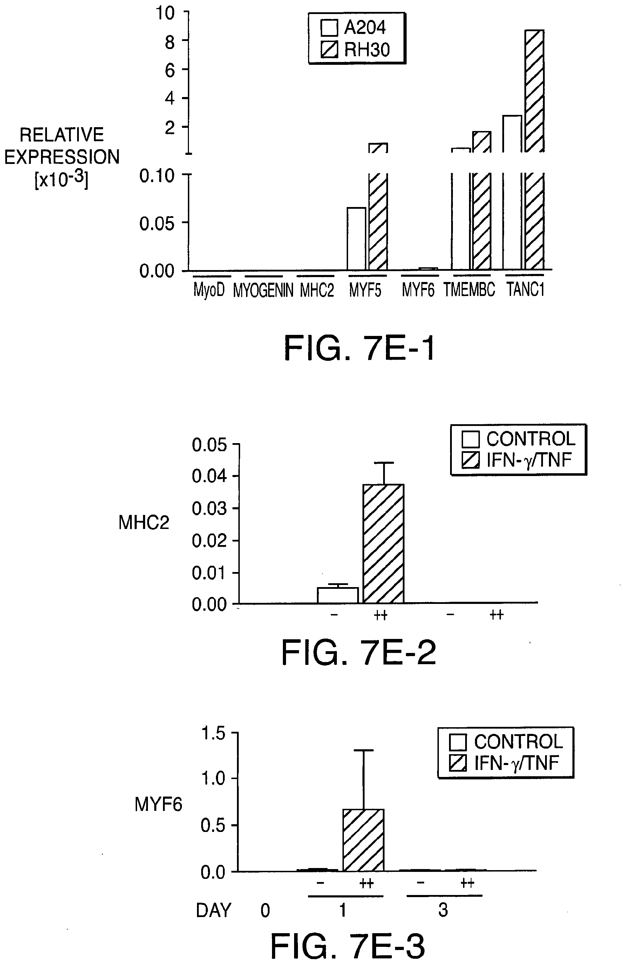

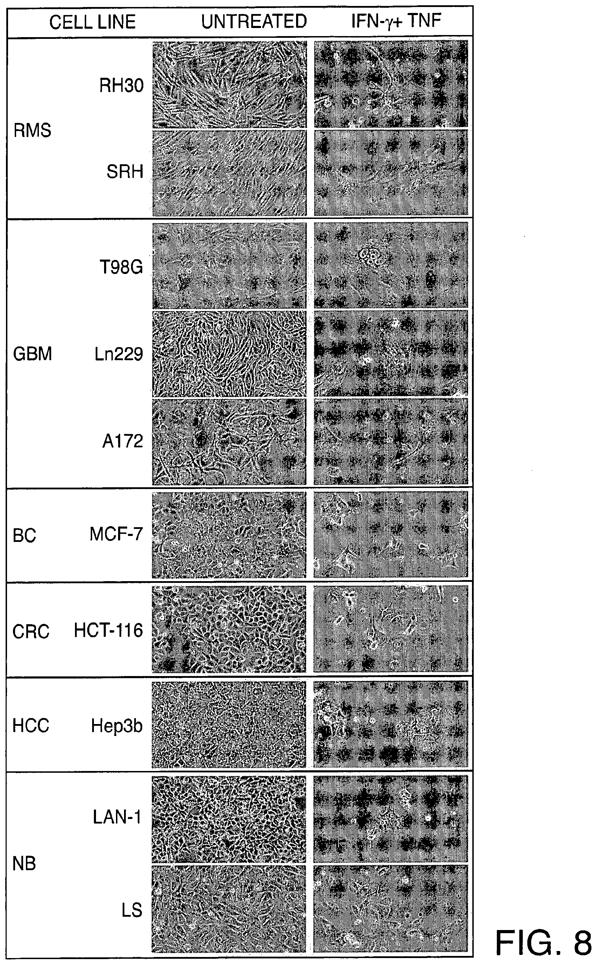

*In vitro,* single application of combined IFN-γ/TNF irrevocably growth arrested A204 sarcoma cells and immediately raised mRNA of cyclin dependent kinase inhibitor p21^{CIP1/WAF1} (7-fold at day +1, 10-fold day5 of untreated control), that in skeletal muscle intrinsicly withdraws the cells from cycling (Figure 7A and 7B). Strikingly, within 5 days IFN-γ/TNF also massively induced bi- and polynucleated cells in sarcoma cultures (>80% of cells polynucleate) (Figure 7C and 7D), with proximal nuclei as seen in late anaphase, suggesting p16^{INK4a}-mediated irreversible block of cytokinesis. In addition, sarcoma cells *de novo* expressed two more muscle-cell-differentiation-regulating markers (Myf-6 and MyosinHeavyChain-II) (Figure 7E), and morphed into SA-β-Galactosidase⁺, elongated multinucleate cells, forming myogenic giant cells (Figure 7C and 7D), a step in advanced muscle cell differentiation. A single administration of IFN-γ/TNF *in vitro* irreversibly growth arrested also RMS cell line RH30 and patient-derived RMS line SRH (passage 14) (Figure 8), but not ZCRH (p16 deficient, passage 9).

### Differentiation-induction in a panel of tumor cell lines respresenting tissue of the three germ layers

To test whether growth arrest and differentiation induced by IFN-γ/TNF may have broader relevance the two T_{H}1 cytokine combinations IFN-y/TNF were tested *in vitro* on a panel of tumor cell lines respresenting tissue of the three germ layers. 3/3 glioblastoma cell lines (T98G, Ln229, A172), 2/2 neuroblastoma cell lines (LS and LAN-1), and cancer cell lines MCF-7 (breast), HCT-116 (colorectal), and Hep3b (hepatocellular carcinoma) stopped proliferation and in unison expressed SA-β-Gal and showed cellular and nuclear atypia of degenerative type (Figure 8). Histopathological characteristics included large hyperchromatic pleomorph nuclei and morphology resembling reactive/non-neoplastic multinucleate myogenic giant cells in skeletal muscle caused by cell injury. In glioblastomas, nuclear atypia resembled that type seen in benign "ancient schwannomas" of peripheral nerves (Figure 8). The development of multinucleate giant cells has been reported in many organs and is linked to infection, injury, autoimmunity and tumor.

### Pharmaceutical compositions

The targeted IL-12 fusion proteins as disclosed herein can be incorporated into a pharmaceutical composition suitable for administration. Such compositions typically comprise the antibody variable regions and a pharmaceutically-acceptable carrier. As used herein the language "pharmaceutically-acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art.

A pharmaceutical composition as disclosed herein is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampules, disposable syringes or multiple dose vials made of glass or plastic.
Medicaments that contain the targeted IL-12 fusion proteins and the IL-7 molecules as described herein which can have a concentration of 0.01 to 100% (w/w), though the amount varies according to the dosage form of the medicaments.

Administration is preferably once per two weeks or once per month, but may be more or less frequent depending on the pharmacokinetic behavior in a given individual. Dosing of the antibody fusion protein as specified in this application for an adult of about 70 kilograms is in the range of about 50 to 1000 milligrams per dose, with a preferred range of about 100 to 500 milligrams per dose. The most preferred dose is about 200 - 400 milligrams for a 70 kg adult treated once per month.

### SHORT DESCRIPTION OF THE FIGURES:

Figure 1 Study design, tumor growth and survival after RMS challenge and therapy. (A) 4-6 week-old NSG mice were irradiated sub-lethally and humanized with CD34⁺ CD3⁻ grafts. Fully engrafted mice were inoculated with 1x10⁶ A204 cells week 12. Immunotherapy began 18 days later when tumor volume was 50-200 mm³. Mice were sacrificed after 5-week treatment, when tumors of FcIL-7 cohort had reached 20% of body weight. Four mice of the NHS-IL12/FcIL-7 and the NHS-IL12/IL-2MAB602 cohort were kept alive and treated at least until day 95 after tumor inoculation. (B) Influence of FcIL-7, NHS-IL12/FcIL-7, and NHS-IL12/IL-2MAB602 on survival. Survival curves were compared using log-rank test. Survival was highly significantly better for NHS-IL12 cohorts compared to FcIL-7 cohort. In the FcIL-7 control group, 4 animals died before day 52 and 3 were sacrificed day 52 due to excessive tumor growth. In the NHS-IL12/FcIL-7 treatment cohort 2 mice died before day 52, 1 on day 56 and 1 on day 74 in the long-term treatment group. (C) Influence of FcIL-7, NHS-IL12/FcIL-7, and NHS-IL12/IL-2MAB602 on tumor growth. Mice bearing human RMS A204 were treated weekly i.v. with FcIL-7 (20 µg) (circles), NHS-IL12 (20 µg)/FcIL-7 (20 µg) (triangles), or with NHS-IL12 (20 µg) and IL-2 (1.5 µg) complexed with MAB602 (15 µg) (squares). Tumor sizes in mm³ indicated as mean ± SD of 7 mice/group treated short-term (5 weeks). (D) Individual tumor sizes of 4 mice per NHS-IL12 group (NHS-IL12/FcIL-7: squares; NHS-IL12/IL-2MAB602: circles) during long-term treatment (14 weeks, >95 days).
Figure 2 : ¹²³I-labelled NHS-IL12 accumulates in the lesion of a human A204 tumor xenograft. (A) *In vivo* SPECT scans performed after 2, 26, and 46 h post injection of therapeutic dose (30 µg) of ¹²³I-labelled NHS-IL12 show specific accumulation of NHS-IL12 in tumor (solid circles) compared to muscle tissue (dotted circles). (B) Uptake of ¹²¹I-NHS-IL12 reached its maximum in the tumor lesion 26 h after administration, whereas in muscle, no specific signal could be detected over the entire scan time. Counts were decay-corrected to adjust for the radioactive decay of ¹²³I between measurement time points (n=2). * *P*≤ 0.05.
Figure 3 Influence of FcIL-7, NHS-IL12/FcIL-7 and NHS-IL12/IL-2MAB602 on innate immunity. (A) Tumor homogenates of individuals in each cohort were tested with RT-PCR-based fragment length analysis for major triggering receptors NKG2C, -D and -E, DNAM-1 and natural killer receptors NKp30, -44 and -46. Note the high congruity within a cohort. (B) Expression of CD161 in homogenates of tumors and muscles. Quantity is given as mean fluorescence intensity. Each dot represents one individual tumor. ** *P*≤ 0.01, *** *P*≤ 0.001, evaluated with one-tailed student's t test. (C) TCR transcripts indicative of iNKT cells (invariant Vα24 and Vβ11), Vδ1 and -2 chains, and NKp46 at day 52. T = tumor tissue, M = muscle tissue, LT = long-term treated mouse day 100, S = single peak, G = Gaussian distribution of Vα24 chain expression. (D) TCRVα24 mRNA expression in A204 tumors detected as a single peak or in Gaussian distribution. In human iNKT TCRVα24 is preferentially associated with a TCRVβ11 chain.
Figure 4 Clonality analysis of αβ T cells under the influence of FcIL-7, NHS-IL12/FcIL-7 and NHS-IL12/IL-2MAB602. (A) Real time PCR-based detection of various immune markers in tumor homogenates. (B) Expression of the target gene was normalized to expression of human CD45. Expression of 25 TRBV segments determined by CDR3-size spectratyping. Filled squares indicate expression of up to 12 fragments. Each vertical lane represents one mouse, dark squares indicate TRBV segments chosen for CDR3 sequence analysis. (C) CDR3 region protein sequences of selected TRBV segments, bold amino acid codes mark homologous sequences. (SEQ ID NO. 6: LDKVGQETQYF; SEQ ID NO. 7: VDRTGEPFYGYTF; SEQ ID NO. 8: STRTGSYNYPLHF; SEQ ID NO. 9: LAEGVTEAFF; SEQ ID NO.10: LAKGVTEAFF; SEQ ID NO. 11: SLNLVLPGGAEAFF; SEQ ID NO. 12: PRFSMNTEAFF; SEQ ID NO. 13: SRGSFESYNEAVLRAQ; SEQ ID NO. 14: SRGSFESYNEQFF; SEQ ID NO. 15: SRGSFESYNEQVLR; SEQ ID NO. 16: RTVANGFSPLHF). (D) T-bet/RORC- and Foxp3/CD40L-expression ratios in tumor homogenates (n=4). * P≤ 0.05, ** P≤ 0.01, *** P≤ 0.001.
Figure 5 Induction of senescence markers and antiproliferative effect by NHS-IL12/FcIL-7 and NHS-IL12/IL-2MAB602 treatment. (A) Cellular senescence and proliferation within tumor sections were determined by immunofluorescence double-staining for nuclear p-HP1γ in combination with PCNA (top panels) or p16^{INK4a} in combination with Ki67 (bottom panels) (1:100). The inserts show a higher magnification of the pictures (1:300) to visualize nuclear dots of p-HP1γ or p16^{INK4a} staining. (B) Mean percentage of p-HP1γ-positive cells (i.e., cells with more than 5 nuclear dots), p16^{INK4a} -positive cells (as determined by higher magnification (1:300)) or Ki67-positive cells after treatment with FcIL-7, NHS-IL12/FcIL-7 or NHS-IL12/IL-2MAB602 (n=3). ** P≤ 0.01, *** P≤ 0.001, evaluated with one-tailed student's t test. (C) Cytokine-induced growth arrest in primary human RMS cancer cell preparations. CCA cells (eRMS, passage 7), SRH (eRMS, passage 8), or ZCRH cells (aRMS, passage > 9) were seeded at a density of 2x10⁴ cells/9.6 cm². Day 3 and 4, cells were treated with 100 ng/ml IFN-γ and 10 ng/ml TNF or medium alone (control). Day 7, cytokines were removed, cells were trypsinized, counted and reseeded at 2x10⁴ cells/9.6 cm². After incubation for another 4 days (ZCRH and SRH) or 10 days (CCA), living cells were counted. Growth curves of the responder cells CCA and SRH and the non-responder cells ZCRH in the absence (Co.) or presence of IFN-γ plus TNF. Mean cell numbers ± SEM (n=3) are shown.
Figure 6 *In vivo* expression and organization of desmin as a marker of myogenic differentiation in A204 RMS. (A) Histologic slides from tumors (n=3/cohort) of all cohorts (FcIL-7, NHS-IL12/FcIL-7, or NHS-IL12/IL-2MAB602 treated) were stained for desmin and (B) were analyzed by a blinded pathologist. LT: long-term treatment; ST: short-term treatment.
Figure 7 Multinucleate, senescent A204 cells and expression of p21 and myogenic markers in native and cytokine-treated A204 cells. (A) Relative expression of p21 before and after treatment with IFN-γ and TNF (++) or medium (-) as a control, measured by quantitative RT-PCR (n=3). (B) IFN-γ and TNF treatment (inverted triangle) terminates cancer cell proliferation but does not kill sarcoma cells (n=3). For comparison, normal cell culture (circles) shows unimpeded proliferation (n=3). (C) upper lane: Cytokine-treated A204 cancer cells are senescent (black arrow: grey staining) and multinucleate (white arrows, DAPI staining). Lower lane: A204 sarcoma cells treated with medium as a negative control are negative for SA-β-Gal and mononuclear (DAPI-staining). (D) Cytokine-induced elongated, multinucleate and syncytial morphology in A204 cells (middle and right) in comparison to A204 cells of standard culture (left), depicted from transmission microscopy. (E) Relative expression of myogenic transcription factors in the A204 and RH30 sarcoma cell line. Induction of MHCII and MYF-6 mRNA expression in A204 cells by 100 ng/ml IFN-γ and 10 ng/ml TNF (++) in comparison to medium (-) over 3 days (n=3).
Figure 8 The cell lines, representing mesodermic, endodermic and ectodermic origin, displayed multinucleate phenotype and growth arrest after treatment with a single dose of 100 ng/ml IFN-γ combined with 10ng/ml TNF (day1) compared to medium control (untreated). Pictures of glioblastoma were taken day 37, all others day 6 after treatment. Cells of all treated cell lines survived day 43, the time point when cells were terminally examined. RMS: rhabdomyosarcoma, GBM: glioblastoma, BC: breast cancer, CRC: colorectal cancer, HCC:hepatocellular carcinoma, NB: neuroblastoma

### MATERIALS AND METHODS:

**(1) Humanization of NSG mice.** HuCD34⁺ stem cells were derived from a surplus of G-CSF mobilized peripheral blood stem cells from parental donors, which have been T-cell depleted by CD34⁺ selection (CliniMACS, Miltenyi, Germany). Cells were suspended 1:2 in a 20% DMSO / 80% 5%-HSA solution and subsequently cryopreserved with a Sylab icecube device and a controlled freezing rate. After thawing, cells were dtained with Trypan Blue and counted in a Neubauer cell count chamber. Informed consent regarding the scientific use of surplus cells was obtained from all donors in accordance with the Declaration of Helsinki. Purity of the CD34⁺ population was further increased to >99.99% by a second round of CD3⁺ depletion after thawing (LS MACS, Miltenyi, Germany). Stem-cell donors were all HLA-mismatched to the RMS A204 cell line. 1x10⁶ huCD34⁺ cells in 100 µl pre-warmed PBS were injected in the tail vein of sub-lethally irradiated (250 cGy) NSG mice. Engraftment was supported by weekly applications of 20 µg FcIL-7 (Merck, Germany). In each of the NHS-IL12 treatment groups, 4 animals received long-term NHS-IL12 cytokine treatment with Fc-IL7 or IL-2MAB602 for a maximum of 15 weeks (100 days).
**(2) Tumor implantation and measure.** Embryonal pediatric RMS A204 cells were obtained from ATCC (HTB 82), thawed, sub-cultured for 1 passage in RPMI 1640+10% FBS, washed three times with sterile saline, tested for mycoplasma with the PCR Mycoplasma Test Kit AppliChem (Germany), and implanted in engrafted NSG mice. Per mouse, 1x10⁶ tumor cells were transplanted in the right flank by s.c. injection. Tumor volume was determined with the following equation: VT = a x b x d x π/6, where a, b and d describe length, width and depth of the tumor. Mice were sacrificed 52 days after tumor inoculation, the time point at which tumors of animals in the FcIL-7 cohorts had reached ≥20% of body weight. Four mice per NHS-IL12 group were further kept alive for long-term treatment.
**(3) Administration of anti-human Histone/humanIL-12 fusion protein and IL-2/anti-IL-2 antibody complexes.** Once per week, 20 µg of the NHS-IL12 fusion protein together with 20 µg FcIL-7 (both from Merck, Germany) was administered via puncture of the tail vein in engrafted humanized NSG mice after tumor had grown to ≥150µl. As stated above, one additional cohort of mice received a mixture of 1.5 µg IL-2 plus 15 µg anti-IL-2 mAB MAB602 per week. Recombinant human IL-2 (PROLEUKIN, Aldesleukin, Chiron, USA) and MAB602 (anti-hIL-2 mABCD122, clone 5355, R&D Systems) were co-incubated for 15 min at room temperature before injection.
**(4) *In Vivo* SPECT/CT Imaging.** *In vivo* imaging of inoculated mice was carried out using an Inveon Multimodality SPECT/CT (Siemens Healthcare, Knoxville, TN, USA). Carrier-free sodium iodide (¹²³I) was purchased from GE Healthcare and radioiodination of NHS-IL12 was performed using Pierce® Iodination Reagent (Thermo Scientific). Mice were injected with 30 µg NHS-IL12 labeled with 18 MBq ¹²³I via the tail vein and *in vivo* SPECT/CT images were acquired 2, 26 and 46 hours after tracer administration. During injection and measurement, mice were anesthetized with 1.5% isoflurane in oxygen (0.5 l/min). Regions of interest (ROI) were contoured on reconstructed SPECT images based on CT information over several slices to cover the entire tumor. For reference, ROIs of equivalent size were placed on unaffected muscle tissue at the left hind leg of the same animal. For evaluation of ¹²¹I-NHS-IL12 uptake, decay corrected counts were used.
**(5) Six-color flow cytometry.** Immune reconstitution was evaluated for several individuals 10-12 weeks after transplantation and peripheral blood, spleen, bone marrow and thymus were analyzed using the following mouse mAbs non-cross-reactive with murine but specific for human epitopes: CD62L(Dreg56)-FITC, CD25(2A3)-APC, CD3(SK7)-PerCP, CD8(SK1)-PerCP, CD8(SK1)-APC-H7, CD4(SK3)-FITC, CD4(SK3)-PerCP, CD14(M5E2)-PE, CD56(My31)-PE, HLA-DR(L243)-PerCp, NKp30(P30-15)-PE, NKp44(P44-8)-APC, NKp46(9E2)-PE as well as their corresponding IgG isotypes (all BD Pharmingen, Germany). CD45(MEM-28)-Pacific Blue, CD19(HIB19)-PerCP, CD3(MEM-57)-Alexa Fluor 700, CD4(MEM-241)-Alexa Fluor 700 (Exbio, Czechoslovakia). CD45(HI30)-PE with respective isotype IgG (Biolegend, Germany). Engraftment was routinely checked 12-14 weeks after transplantation by retro-orbital bleeding and FACS staining. A204 cell line was characterized with ULBP-1(Z-9), ULBP-2(2F9), ULBP-3(F16), ULBP-4(6E6) (all Santa Cruz, USA), MICA/B(6D4)APC CD112(R2.525)PE, CD155(SKII.4)PE (all Biolegend, Germany), HLA-ABC(W6/32)PE (DAKO Cytomation, Germany), secondary antibody RAM-PE(X56) (BD Pharmingen, Germany), and isotype controls (Beckman Coulter and R&D Systems, Germany). Flow cytometry was performed on an LSR II (BD Biosciences) using Diva© software.
**(6) Immunohistology staining.** Frozen tissue slides (5 µm thickness) were incubated in 4% buffered formalin (2 min), washed in *aqua dest*., boiled in citrate buffer pH 6.0 in a pressure chamber (4 min), and washed in Tris-NaCI-Tween. Sample slides were transferred to a wet chamber and stained with Zytochem-Plus AP Polymer-Kit (Zytomed Systems, Germany). Primary antibodies were: CD3 (SP7, 1:50; DCS Innovative Diagnostic Systeme GmBH, Germany), monoclonal rabbit anti human CD4 (SP35, 1:50, Zytomed Systems), CD8 (C8/144B, 1:100), CD56 (123C3-D5, 1:20), CD68 (PG-M1, 1:150), HLA-DR-α (TAL.1B5, 1:200), desmin (D33, 1:100, all DAKO, Germany), Perforin (5810, 1:200, Novocastra/Leica, Germany). Final staining was performed with Permanent AP Red Kit (Zytomed Systems, Germany). Single-blinding was performed for analysis of immunohistological slides.
**(7) Immunofluorescence.** Fresh frozen cryostat sections of human xenografted A204 tumors were stained as described previously (Zhang and Adams (2007) Cell Cycle 6:784-789). Briefly, cryostat sections were fixed with periodate-lysine-paraformaldehyde and blocked with donkey serum (1:20) for 30 min at room temperature (RT). Slides were then incubated with rabbit-anti-p-HP1γ (1:80; Abcam, UK), mouse-anti-PCNA (1:50; Cell Signaling Technology, USA), mouse-anti-p16^{INK4a} (1:50; Santa Cruz Biotechnology, Germany), or rabbit-anti-Ki67 (1:100; Abcam, USA) for 1h, RT. After 3 washes, sections were incubated with Cy3- or Cy5-conjugated donkey-anti-rabbit antibody and Cy3- or Cy5-conjugated donkey-anti-mouse antibody (all Dianova, Germany). Before mounting slides with Mowiol (Hoechst, Germany), nuclei were stained with Yopro (1:2000; Invitrogen, Germany) (5 min), and sections analyzed with a Leica TCS-Sp/Leica DM RB confocal laser scanning microscope (Leica Microsystems, Germany). Images were processed with the Leica Confocal Software LCS (Version 2.61).
**(8) Treatment of patient-derived primary RMS and cancer cell lines with TNF and IFN-**γ. Permanent growth arrest of different cancer cell lines (rhabdomyosarcomas RH30, SRH; glioblastomas T98G, Ln229, A172; breast cancer MCF-7; colorectal cancer HCT-116; hepatocellular carcinoma Hep3b and neurblastomas LAN-1, LS) after cytokine treatment was determined using the procedure exactly described earlier (Braumuller et al., 2013, Nature 494:361-365). Short-term culture to study early differentiation events in RMS was accomplished by seeding tumor cells in low numbers (4.000 or 8.000 cells/cm²) and incubating with medium with and without 10 ng/ml TNF + 100 ng/ml IFN-γ.
**(8) KIR expression analysis.** Expression analysis was performed as previously described (77). NKp30, -44, -46, DNAM-1, CD161 transcripts were determined with specific primers in end-point PCRs using 5'FAM-labelled reverse primers. PCR products were analyzed for fragment length in an ABI sequencer with Gene Scan-600 LIZ for length standard and GeneMapper software (both Applied Biosystems, Germany); MFI was used for semiquantitative analysis.
**(9) Expression of T-bet, RORC, Foxp3, CD40L, granzyme B, granzyme K.** Intratumoral gene expression was determined with real-time PCR on a BioRad C1000 Thermal cycler/CFX96 real-time System (Munich, Germany), using cDNA specific primers (all sequences available on request) and the iQ SYBR Green Supermix BioRad (Munich, Germany). Expression was normalized to human CD45 expression. RORC expression was detected with a specific primer set (Search LC, Germany) and FastStart DNA SYBR Green I (Roche, Germany).
**(10) Vα-24 chain identification.** For this the protocol published by Han et al. (Han et al, 1999, J. Immunol. 163:301-31) was used.
**(11) Vβ spectratyping analysis.** Diversity of the TCR Vβ-chain expression and complexity of TCR repertoire was analyzed according to Gorski et al. (Gorsk et al., 1994, J. Immunol. 152:5109-511) with minor modifications. 5'FAM-labelled Cβ-primer was used, and an ABI sequencer with Gene Scan-600 LIZ and GeneMapper software for detection of amplicons (both Applied Biosystems, Germany).
**(12) γδ immunoscope.** The immunoscope was determined as published previously (Dechanet et al., ,J., 1999, J. Clin. Invest 103:1437-1449).
**(13) Identification of TCR-CDR3 regions.** Vβ PCR products were cloned into pGEMTeasy (Promega, Germany) and amplified in XL1-Blue competent cells (Stratagene, USA) using standard procedures. Insert-positive clones were conveyed directly to a reamplifying Vβ PCR. 5µl PCR aliquots were analyzed on a 2.5% agarose gel, and PCR products of relevant length sent to Seqlab, Germany for sequence analysis. Translation of cDNA into protein sequence was conducted with EMBOSS Transeq free software.

## Claims

1. Tumor-targeted IL-12 for use in combination treatment with IL-7 for inducing and / or stimulating the immune response against a cancer disease in a patient suffering from said cancer disease, wherein said induction or stimulation causes
(i) senescence or growth arrest of cancer cells, and / or
(ii) remission of cancer cells or cancer tissue to cells or tissue of origin,
caused by said drug,
and wherein the tumor-targeted IL12 is fused via the N-terminus of its p35 subunit to the C-terminus of the fully human NHS76 antibody or biological effective portion thereof which is designated as NHS-IL-12 and wherein said IL-7 is covalently fused to the C-terminus of the Fc fragment of an immunoglobulin.

2. Tumor-targeted IL-12 for use in combination treatment with IL-7 according to claim 1, wherein said cancer cell senescence is caused by generating endogenous IFNγ and / or TNF in succession of said stimulation or induction of the patient's immune system triggered by said drug.

3. Tumor-targeted IL-12 for use in combination treatment with IL-7 according to claim 1 or claim 2, wherein the senescence of the cancer cells results in stable growth arrest.

4. Tumor-targeted IL-12 for use in combination treatment with IL-7 according to any of claims 1 - 3, wherein the senescence of the cancer cells is independent on direct immune specific cytotoxic effects.

5. Tumor-targeted IL-12 for use in combination treatment with IL-7 according to any of claims 1 - 4, wherein the cancer disease is related to solid tumors, or tumors of the muscle, bone, nerves, cartilage, tendons, blood vessels, and fatty or fibrous tissues.

6. Tumor-targeted IL-12 for use in combination treatment with IL-7 according to claim 5, wherein the cancer is sarcoma.

7. Tumor-targeted IL-12 for use in combination treatment with IL-7 according to claim 5 or 6, wherein the therapy initiates induction of myogenic differentiation.

8. Tumor-targeted IL-12 for use in combination treatment with IL-7 according to any of claims 1-7 in combination with radiotherapy or radio-chemotherapy.

9. Medicament containing tumor-targeted NHS-IL12 and FcIL-7 in a concentration of 0.01 to 100% (w/w).

## Patentansprüche

1. Gegen Tumore gerichtetes IL-12 zur Verwendung bei einer Kombinationsbehandlung mit IL-7 zum Induzieren und/oder Stimulieren der Immunantwort gegen eine Krebserkrankung bei einem Patienten, der an der Krebserkrankung leidet, wobei die Induktion oder Stimulation Folgendes bewirkt:
(i) Seneszenz oder Wachstumshemmung der Krebszellen und/oder
(ii) Remission der Krebszellen oder des Krebsgewebes zu Ursprungszellen oder -gewebe, die durch den Arzneistoff bewirkt wird,
und wobei das gegen Tumore gerichtetes IL12 über den N-Terminus seiner p35-Untereinheit an den C-Terminus des vollständig humanen Antikörpers NHS76 oder einen wirksamen Abschnitt davon fusioniert ist, was als NHS-IL-12 bezeichnet wird, und wobei das IL-7 kovalent an den C-Terminus des Fc-Fragments eines Immunglobulins fusioniert ist.

2. Gegen Tumore gerichtetes IL-12 zur Verwendung bei einer Kombinationsbehandlung mit IL-7 nach Anspruch 1, wobei die Krebszellseneszenz durch Erzeugen von endogenem IFNγ und/oder TNF kurz nach der durch den Arzneistoff ausgelösten Stimulation oder Induktion des Immunsystems des Patienten bewirkt wird.

3. Gegen Tumore gerichtetes IL-12 zur Verwendung bei einer Kombinationsbehandlung mit IL-7 nach Anspruch 1 oder Anspruch 2, wobei die Seneszenz der Krebszellen zu einer stabilen Wachstumshemmung führt.

4. Gegen Tumore gerichtetes IL-12 zur Verwendung bei einer Kombinationsbehandlung mit IL-7 nach einem der Ansprüche 1-3, wobei die Seneszenz der Krebszellen unabhängig von direkten immunspezifischen zytotoxischen Wirkungen ist.

5. Gegen Tumore gerichtetes IL-12 zur Verwendung bei einer Kombinationsbehandlung mit IL-7 nach einem der Ansprüche 1-4, wobei die Krebserkrankung mit soliden Tumoren oder Tumoren des Muskels, des Knochens, der Nerven, des Knorpels, der Sehnen, der Blutgefäße und der Fett- oder Bindegewebe in Zusammenhang steht.

6. Gegen Tumore gerichtetes IL-12 zur Verwendung bei einer Kombinationsbehandlung mit IL-7 nach Anspruch 5, wobei es sich bei dem Krebs um Sarkom handelt.

7. Gegen Tumore gerichtetes IL-12 zur Verwendung bei einer Kombinationsbehandlung mit IL-7 nach Anspruch 5 oder 6, wobei die Therapie die Induktion der myogenen Differenzierung initiiert.

8. Gegen Tumore gerichtetes IL-12 zur Verwendung bei einer Kombinationsbehandlung mit IL-7 nach einem der Ansprüche 1-7 in Kombination mit Radiotherapie oder Radiochemotherapie.

9. Arzneimittel, das gegen Tumore gerichtetes NHS-IL12 und FcIL-7 in einer Konzentration von 0,01 bis 100% (Gew./Gew.) enthält.

## Revendications

1. IL-12 à ciblage tumoral pour une utilisation dans un traitement associatif avec IL-7, pour l'induction et/ou la stimulation de la réponse immunitaire contre une maladie cancéreuse chez un patient souffrant de ladite maladie cancéreuse, où ladite induction ou stimulation provoque
(i) une sénescence ou un arrêt de croissance des cellules cancéreuses, et/ou
(ii) une rémission des cellules cancéreuses ou du tissu cancéreux vers des cellules ou du tissu d'origine,
provoquées par ledit médicament,
et où l'IL-12 à ciblage tumoral est fusionnée via l'extrémité N-terminale de sa sous-unité p35 à l'extrémité C-terminale de l'anticorps NHS76 totalement humain ou d'une portion efficace biologique de celui-ci qui est désigné par NHS-IL-12 et où ladite IL-7 est fusionnée de manière covalente à l'extrémité C-terminale du fragment Fc d'une immunoglobuline.

2. IL-12 à ciblage tumoral pour une utilisation dans un traitement associatif avec IL-7 selon la revendication 1, où ladite sénescence des cellules cancéreuses est provoquée par la génération d'IFNγ et/ou de TNF endogène suite à ladite stimulation ou induction du système immunitaire du patient déclenchée par ledit médicament.

3. IL-12 à ciblage tumoral pour une utilisation dans un traitement associatif avec IL-7 selon la revendication 1 ou la revendication 2, où la sénescence des cellules cancéreuses entraîne un arrêt de croissance stable.

4. IL-12 à ciblage tumoral pour une utilisation dans un traitement associatif avec IL-7 selon l'une quelconque des revendications 1-3, où la sénescence des cellules cancéreuses est indépendante d'effets cytotoxiques spécifiques immunitaires directs.

5. IL-12 à ciblage tumoral pour une utilisation dans un traitement associatif avec IL-7 selon l'une quelconque des revendications 1-4, où la maladie cancéreuse est liée aux tumeurs solides, ou aux tumeurs des muscles, des os, des nerfs, du cartilage, des tendons, des vaisseaux sanguins, et aux tissus gras ou fibreux.

6. IL-12 à ciblage tumoral pour une utilisation dans un traitement associatif avec IL-7 selon la revendication 5, où le cancer est un sarcome.

7. IL-12 à ciblage tumoral pour une utilisation dans un traitement associatif avec IL-7 selon la revendication 5 ou 6, où la thérapie initie l'induction d'une différentiation myogénique.

8. IL-12 à ciblage tumoral pour une utilisation dans un traitement associatif avec IL-7 selon l'une quelconque des revendications 1-7, en combinaison avec une radiothérapie ou une radio-chimiothérapie.

9. Médicament contenant du NHS-IL-12 et du Fc-IL-7 à ciblage tumoral selon une concentration allant de 0,01 à 100% (p/p).
